# EUROPEAN PATENT APPLICATION

(11) **EP 4 129 301 A1**
(43) Date of publication of application: **08.02.2023**
(21) Application number: 21784142.8
(22) Date of filing: 06.04.2021
(51) Int. Cl.: A61K 31/59, A61K 31/593, A61P 11/00

(54) **COMPOUNDS FOR THE PROPHYLAXIS AND/OR TREATMENT OF ACUTE RESPIRATORY DISTRESS SYNDROME**

(30) Priority: 06.04.2020 ES 202030281
(71) Applicant: Servicio Andaluz de Salud, 41071 Sevilla (ES)
(72) Inventor: QUESADA GÓMEZ, José Manuel, 41071 Sevilla (ES); ENTRENAS COSTA, Luis Manuel, 41071 Sevilla (ES); NAVARRO VALVERDE, Cristina, 41071 Sevilla (ES)
(74) Representative: Hoffmann Eitle
(86) International application number: PCT/ES2021/070228
(87) International publication number: WO 2021/205049

(57) **Abstract**

The invention relates to calcifediol for the prophylaxis and/or treatment of acute respiratory distress syndrome.

## Description

### FIELD OF THE INVENTION

The present invention is comprised within the field of medicine and relates to the use of vitamin D receptor (VDR) stimulators and/or agonists for the prophylaxis and/or treatment of acute respiratory distress syndrome, and specifically of severe acute respiratory syndrome coronavirus 2 (SARS-CoV-2).

### PRIOR ART

At the end of December 2019, patients with a cough, fever, and dyspnea with severe acute respiratory syndrome (SARS) due to an unidentified microbial infection were reported in Wuhan, China. The epidemic spread of this syndrome has grown from the time it emerged to where it is today.

The new pneumonia induced by coronavirus was officially named coronavirus disease 2019 (COVID-19) by the WHO on 11 February 2020, while the International Committee on Taxonomy of Viruses announced that the new coronavirus was called severe acute respiratory syndrome coronavirus 2 (SARS-CoV-2).

Coronaviruses form spherical enveloped particles 100-160 nm in diameter. They contain a positive-sense, single-stranded RNA (ssRNA) genome 26-32 kb in size. Four coronavirus genera (α, β, γ, δ) have been identified to date, with human coronaviruses (HCoV) detected in the coronavirus α (HCoV-229E and NL63) and coronavirus β (MERS-CoV, SARS-CoV, HCoV-OC43, and HCoV-HKU1) genera.

In addition to COVID-19, coronaviruses have previously caused two other epidemic diseases: severe acute respiratory syndrome (SARS) and Middle East respiratory syndrome (MERS). COVID-19 is currently considered a global pandemic. On 31 January 2020, the World Health Organization (WHO) announced that COVID-19 was listed as a Public Health Emergency of International Concern (PHEIC), which means that it may involve risks for a number of countries and requires a powerful and organized national and international coordination in order to respond.

Patients with COVID-19 show clinical manifestations including fever, unproductive cough, dyspnea, myalgia, fatigue, normal or lowered white blood cell counts, and radiographic evidence of pneumonia, which are similar to the symptoms of SARS-CoV and MERS-CoV infections. Therefore, despite the fact that little is known about COVID-19 pathogenesis, similar mechanisms of SARS-CoV and MERS-CoV can still offer a great deal of information about the SARS-CoV-2 infection pathogenesis in order to facilitate learning about COVID-19 pathogenesis). Since it was first described, the main cause of death of COVID-19 was known to be acute respiratory distress syndrome (ARDS). ARDS is the immunopathological event common for SARS-CoV-2, SARS-CoV, and MERS-CoV infections. One of the main mechanisms for ARDS is the so-called cytokine storm, the uncontrolled fatal systemic inflammatory response resulting from the release of large amounts of proinflammatory cytokines (IFN-α, IFN-γ, IL-1β, IL-6, IL-12, IL-18, IL-33, TNF-α, TGFβ, etc.) and chemokines (CCL2, CCL3, CCL5, CXCL8, CXCL9, CXCL10, etc.) by immune effector cells in SARS-CoV infection.

Similar to those with SARS-CoV, patients with severe MERS-CoV infection show elevated serum levels of IL-6, IFN-α, and CCL5, CXCL8, CXCL-10 compared with those patients having mild-moderate disease.

Furthermore, the excessive activation and recruitment of activated neutrophils in inflamed lungs exacerbate ARDS pathogenesis and may indicate a poor clinical outcome. The migration of activated neutrophils to the lung is mediated by several factors, among which chemokines and cell adhesion molecules are considered the most important. CXC motif chemokine 10 (CXCL10), also known as interferon gamma-induced protein 10 (IP-10) (CXCL10), is a chemokine that modulates innate and adaptive immune responses by means of recruiting inflammatory cells (i.e., neutrophils, T-lymphocytes, and NK cells) at inflammation sites. Upon binding to its receptor, CXCR3, CXCL10 can induce chemotaxis, apoptosis, cell growth, and angiostasis. Patients infected with ARDS also show unusually high levels of CXCL10, and uncontrolled inflammation is associated with the development of ARDS

In summary, the cytokine storm will trigger a violent attack on the body by the immune system, will cause ARDS and multiorgan failure, and will lastly lead to death in severe cases of SARS-CoV-2 infection, as occurs in SARS-CoV and MERS-CoV infections. Patients infected with 2019-nCoV also showed higher amounts of IL1 B, IFNγ, IP10 and monocyte chemoattractant protein-1 (MCP-1), which probably brough about activated T-helper-1 (Th1) cell responses.

Furthermore, patients who required being admitted to the ICU showed higher concentrations of GCSF, IP10, MCP1, MIP1A, and TNFα than those who did not require being admitted to the ICU, which suggests that the cytokine storm is associated with the severity of the disease.

Acute respiratory distress syndrome (ARDS) is a life-threatening overwhelming condition characterized by endothelial disruption and increased permeability of the endothelial-alveolar barrier, in which the lungs are inflamed and damaged. In this state, the lungs cannot transfer enough oxygen to the blood for the vital organs of the body. It is generally observed in patients who are already gravely ill. This virulent systemic inflammatory process is associated with significant morbidity and mortality.

The treatment of ARDS involves general support measures necessary for all critical patients (for example, control of infections, early enteral nutrition, prophylaxis of stress-induced ulcers and thromboprophylaxis) combined with various ventilation strategies

There are currently no specific effective therapeutic options available for this condition.

Neuromuscular agents (NMAs) can be used to improve the synchrony of the patient's ventilator and to help with mechanical ventilation in patients with severe hypoxemia. There is evidence that the use of NMAs in patients with severe ARDS (PaO₂ / FiO₂ <150 mm Hg) improves oxygenation and reduces inflammatory cytokine.

Inhaled nitric oxide (NO) is an endogenous vasodilator. When inhaled, it reduces the V/Q mismatch and improves oxygenation by means of selective pulmonary vasodilation in alveolar units which are ventilated. Inhaled NO also reduces elevated pulmonary vascular resistance in patients with ARDS. The adverse effects of inhaled NO are methemoglobinemia, cytotoxic nitrogen products (nitrogen dioxide), and kidney failure. A review by Cochrane of 14 clinical trials with 1303 patients (which included three pediatric studies and one combined study for adults and children) showed only a temporary improvement in oxygenation without the benefit of survival or an increase in days off a ventilator. Furthermore, improvements in the secondary outcomes, such as the duration in ICU or the hospital stay were not observed, and increased kidney failure was observed in the group treated with inhaled NO. Current use is decreasing due to the data on deficient outcomes and the growing costs of the use of inhaled NO. Use thereof is not recommended as routine therapy, but it could be considered to improve oxygenation in patients with refractory hypoxemia.

Prostacyclins are arachidonic acid derivatives which cause pulmonary vasodilation and are used to treat patients with primary pulmonary hypertension. They have further immunomodulatory effects, such as reducing neutrophil adhesion and inhibiting neutrophil, macrophage, and platelet activation. Nebulized prostacyclin (PGI2) presents comparable effects in improving oxygenation and pulmonary vasodilation, and in reducing bypass compared with inhaled NO. Clinically speaking, prostanoids are rarely used and are not recommended for routine practice.

Ketoconazole is an imidazole-based antifungal medicinal product which inhibits synthesis of thromboxane A2, a potent vasoconstrictor involved in platelet aggregation and neutrophil recruitment. It is also known to reduce the alveolar macrophage inflammatory mediator, and its role as an antiinflammatory agent in ARDS was therefore evaluated. Although the earliest preventive studies suggested benefits, a phase III study conducted in 2000 by the ARDS Network did not show any improvement in mortality or in secondary outcome measures.

Pentoxifylline is a phosphodiesterase inhibitor, and lisofylline is a pentoxifylline derivative with antiinflammatory properties. Lisofylline reduces elevated circulating levels of oxidized free fatty acids, which are observed in patients with ARDS, and inhibits neutrophil accumulation, in addition to reducing proinflammatory cytokines (TNFa, IL1, and IL6).

Oxygen free radicals produced by activated neutrophils and macrophages are thought to play an important role in the inflammatory pathways leading to cell damage in patients with ARDS. Glutathione is an antioxidant produced in the liver, the levels of which are reduced in the alveolar liquid in patients with ARDS. Glutathione levels can be restored by means of supplementation with the precursor thereof, N-acetylcysteine. Several small studies have not shown any mortality benefits with the use of N-acetylcysteine in patients with ALI and ARDS.

ARDS is characterized by a significant inflammatory process followed by fibroproliferative changes. The use of steroids to reduce this inflammation or to moderate fibrotic recovery is an obvious approach that has been tested in several clinical studies. The corticosteroid dose, treatment duration, and the start time of both in the prevention and in the treatment of the ARDS have been evaluated. However, current evidence in patients with SARS and MERS suggests that receiving corticosteroids had no effect on mortality, but rather delayed viral clearance.

According to a recent systematic review (Lan et al. Sci Rep. 2020 Aug 31;10(1):14261. doi: 10.1038/s41598-020-71271-9), the administration of vitamin D did not provide further advantages over placebo for critically ill patients. Nine clinical trials which included 1640 critically ill patients were reviewed. Vitamin D was administered enterally in five studies, by means of intravenous or intramuscular injection in three studies, and both ways in one study. Calcitriol was used in only one of the reviewed clinical trials, and cholecalciferol (vitamin D3) was used in the rest. This review only included randomized controlled trials (RCTs) which compared the efficacy of supplementation with vitamin D to placebo in critically ill adult patients. The primary outcome was mortality at 28 days. In the grouped analysis of the 9 RCTs, no significant differences were observed in mortality at 28 days between the vitamin D supplementation and placebo groups (20.4% vs 21.7%, OR, 0.73; 95% Cl, 0.46-1.15; I 2 = 51%). This outcome did not vary according to the method of vitamin D supplementation (only enterally: 19.9% vs 18.2%, OR, 1.19; 95% Cl, 0.88-1.57; I 2 = 10%; by intramuscular or intravenous injection: 25.6% vs 40.8%, OR, 0.48; CI 95%, 0.21-1.06; I 2 = 19%), or daily dose (high dose: 20.9% vs 19.8%, OR, 0.83; CI 95%, 0.51-1.36; I 2 = 53%; low dose: 15.6% vs 21.3%, OR, 0.74; 95% Cl, 0.32-1.68; I 2 = 0%). No significant differences were observed between the vitamin D supplementation and placebo groups with respect to the duration of the stay in the ICU (standard mean difference [SMD], - 0.30; 95% Cl, - 0.61 to 0.01; I 2 = 60%), duration of hospital stay (SMD, - 0.17; 95% Cl, - 041 to 0.08; I 2 = 65%) and duration of mechanical ventilation (SMD, - 0.41; 95% Cl, - 081 to 0.00 ; I 2 = 72%).

Other reviews and even recent papers conclude the same, that is, no significant differences are observed between the vitamin D supplementation and placebo groups.

The authors of the present invention have demonstrated by means of a randomized clinical trial that, unlike the absence of efficacy obtained with the administration of cholecalciferol or calcitriol, the administration of calcifediol in a given dosage regimen produces clinically and statistically significant differences between patients who were administered calcifediol in addition to the best available treatment and those who were administered only the best available treatment.

### DESCRIPTION OF THE INVENTION

The authors of the present invention propose an alternative therapeutic approach with respect to the current approach. The previously conducted trials use cholecalciferol, but not calcifediol. Calcifediol is the pre-hormone of the VDES, the substrate for the synthesis of 1,25(OH)2D or calcitriol by means of the action of 1α hydroxylase (CYP2721B) both in the kidney due to its endocrine actions, and in multiple cells of the organism due to its auto/paracrine action. Calcitriol acts as a hormone and binds with a high affinity to its nuclear receptor, vitamin D receptor (VDR), regulating the transcription of a large number (~3%) of genes, with a broad spectrum of functional activities. Calcitriol, also known as 1,25-dihydroxy-vitamin D3 (1,25-(OH)2D3), is the biologically hormonal form of the vitamin D endocrine system, and it exerts its biological actions through stimulation of the specific vitamin D receptor (VDR). The outcome obtained by the authors with the use of calcifediol in patients with SARS-CoV-2 is surprising, not only because it is superior to what is deduced from the earlier systematic reviews of trials in which other forms of vitamin D have been used, but also because it is superior to any other treatment described, with the exception of vaccines.

### MEDICAL USES OF THE COMPOUNDS OF THE INVENTION

Therefore, a **first aspect** of the invention relates to the use of a vitamin D receptor (VDR) agonist for the prevention, improvement, mitigation, or treatment of acute respiratory distress syndrome (ARDS).

In a preferred embodiment of this aspect of the invention, the vitamin D receptor (VDR) agonist is a compound of formula (I): 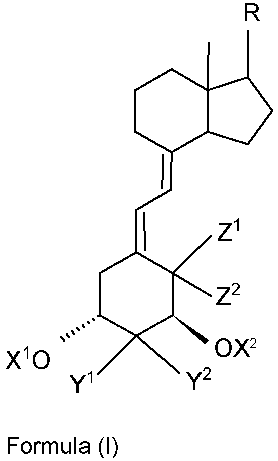 or any of the pharmaceutically acceptable salts, esters, tautomers, solvates, and hydrates thereof, or any combinations thereof, wherein X¹ and X² are each selected from the group consisting of hydrogen and acyl; wherein Y¹ and Y² can be H, or one can be O-aryl or O-alkyl while the other one is hydrogen and can have a β or α configuration; Z¹ and Z² are both H, or Z¹ and Z² taken together are CH2; and wherein R is an alkyl, hydroxyalkyl, or fluoroalkyl group, or R can represent the following side chain: 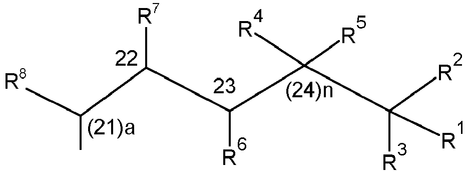 wherein (a) can have an S or R configuration, and wherein R¹ represents hydrogen, hydroxy, or O-acyl, R² and R³ are each selected from the group consisting of alkyl, hydroxyalkyl, and fluoroalkyl or, when taken together, represent the group ~(CH2)m-: where m is an integer having a value of 2 to 5, R4 is selected from the group consisting of hydrogen, hydroxy, fluorine, O-acyl, alkyl, hydroxyalkyl, and fluoroalkyl, R5 is selected from the group consisting of hydrogen, hydroxy, fluorine, alkyl, hydroxyalkyl, and fluoroalkyl, or R4 and R5 taken together represent double-bonded oxygen, R6 and R7 taken together form a carbon-carbon double bond, and R8 can be H or CH3, and wherein n is an integer having a value of 1 to 5, and wherein the carbon at any of positions 20, 22, or 23 in the side chain can be replaced with an O, S, or N atom,
or any of the pharmaceutically acceptable salts, esters, tautomers, solvates, and hydrates thereof, or any combinations thereof, for the prevention, improvement, mitigation, or treatment of acute respiratory distress syndrome (ARDS).

In another preferred embodiment of the invention, the compound is calcifediol, calcidiol, 25-hydroxycholecalciferol, or 25-hydroxyvitamin D (abbreviated as 25(OH)D), of formula (II), and CAS number: 19356-17-3, or any of the pharmaceutically acceptable salts, esters, tautomers, solvates, and hydrates thereof, or any combinations thereof:

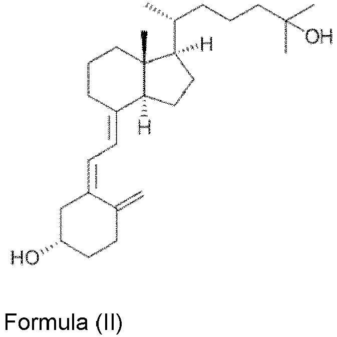

In another preferred embodiment of the invention, the compound is calcitriol, 1-alpha,25-dihydroxycholecalciferol (abbreviated as 1,25-(OH)2D3), of formula (III), and CAS number: 32222-06-3, or any of the pharmaceutically acceptable salts, esters, tautomers, solvates, and hydrates thereof, or any combinations thereof.

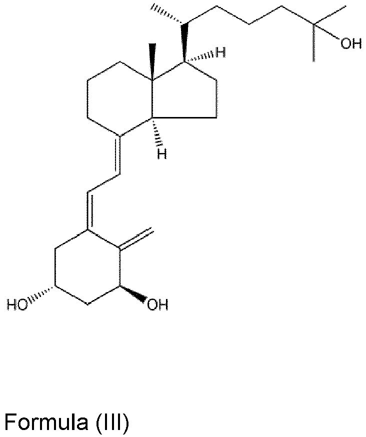

In another preferred embodiment of the invention, the compound is paricalcitol, 19-nor-1,25-(OH)2-vitamin D2, of formula (IV), and CAS number: 131918-61-1, or any of the pharmaceutically acceptable salts, esters, tautomers, solvates, and hydrates thereof, or any combinations thereof.

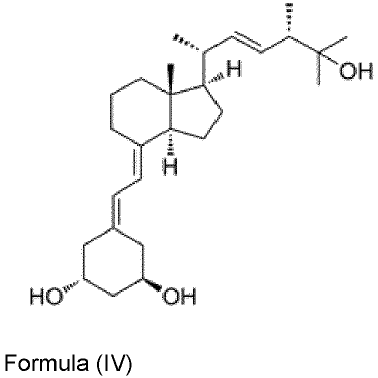

The vitamin D receptor (VDR, or also NR1I1 or PPP1R163) is encoded by a gene that is a member of the superfamily of nuclear hormone receptors of ligand-inducible transcription factors. This receptor also works as a receptor for the secondary bile acid, lithocholic acid. The downstream targets of the vitamin D3 receptor are mainly involved in mineral metabolism, although this receptor regulates a variety of other metabolic pathways. Mutations in this gene are associated with vitamin D-resistant rickets type II. A single-nucleotide polymorphism in the start codon results in an alternative translation start site three codons downstream. Alternatively, spliced transcription variants encoding different isoforms for this gene have been described.

In the context of the present invention, VDR is also defined by a nucleotide or polynucleotide sequence, which constitutes the coding sequence of protein NK-1R, and would comprise different variants from:
a) nucleic acid molecules encoding a polypeptide comprising the amino acid sequence of SEQ ID NO: 1,
b) nucleic acid molecules the complementary chain of which hybridizes with the polynucleotide sequence of a),
c) nucleic acid molecules the sequence of which differs from a) and/or b) due to the degeneration of the genetic code,
d) nucleic acid molecules encoding a polypeptide comprising the amino acid sequence with an identity of at least 80%, 90%, 95%, 98%, or 99% to SEQ ID NO: 1, wherein the polypeptide encoded by said nucleic acids presents the activity and the structural characteristics of VDR.

SEQ ID NO: 1
SEQ ID NO: 2

GGGAGCGCGGAACAGCTTGTCCACCCGCCGGCCGGACCAGGGATGGAGGCAAT
GGCGGCCAGCACTTCCCTGCCTGACCCTGGAGACTTTGACCGGAACGTGCCCCG
GATCTGTGGGGTGTGTGGAGACCGAGCCACTGGCTTTCACTTCAATGCTATGACC
TGTGAAGGCTGCAAAGGCTTCTTCAGGCGAAGCATGAAGCGGAAGGCACTATTCA
CCTGCCCCTTCAACGGGGACTGCCGCATCACCAAGGACAACCGACGCCACTGCC
AGGCCTGCCGGCTCAAACGCTGTGTGGACATCGGCATGATGAAGGAGTTCATTCT
GACAGATGAGGAAGTGCAGAGGAAGCGGGAGATGATCCTGAAGCGGAAGGAGG
AGGAGGCCTTGAAGGACAGTCTGCGGCCCAAGCTGTCTGAGGAGCAGCAGCGCA
TCATTGCCATACTGCTGGACGCCCACCATAAGACCTACGACCCCACCTACTCCGA
CTTCTGCCAGTTCCGGCCTCCAGTTCGTGTGAATGATGGTGGAGGGAGCCATCCT
TCCAGGCCCAACTCCAGACACACTCCCAGCTTCTCTGGGGACTCCTCCTCCTCCT
GCTCAGATCACTGTATCACCTCTTCAGACATGATGGACTCGTCCAGCTTCTCCAAT
CTGGATCTGAGTGAAGAAGATTCAGATGACCCTTCTGTGACCCTAGAGCTGTCCC
AGCTCTCCATGCTGCCCCACCTGGCTGACCTGGTCAGTTACAGCATCCAAAAGGT
CATTGGCTTTGCTAAGATGATACCAGGATTCAGAGACCTCACCTCTGAGGACCAG
ATCGTACTGCTGAAGTCAAGTGCCATTGAGGTCATCATGTTGCGCTCCAATGAGT
CCTTCACCATGGACGACATGTCCTGGACCTGTGGCAACCAAGACTACAAGTACCG
CGTCAGTGACGTGACCAAAGCCGGACACAGCCTGGAGCTGATTGAGCCCCTCAT
CAAGTTCCAGGTGGGACTGAAGAAGCTGAACTTGCATGAGGAGGAGCATGTCCT
GCTCATGGCCATCTGCATCGTCTCCCCAGATCGTCCTGGGGTGCAGGACGCCGC
GCTGATTGAGGCCATCCAGGACCGCCTGTCCAACACACTGCAGACGTACATCCG
CTGCCGCCACCCGCCCCCGGGCAGCCACCTGCTCTATGCCAAGATGATCCAGAA
GCTAGCCGACCTGCGCAGCCTCAATGAGGAGCACTCCAAGCAGTACCGCTGCCT
CTCCTTCCAGCCTGAGTGCAGCATGAAGCTAACGCCCCTTGTGCTCGAAGTGTTT
GGCAATGAGATCTCCTGACTAGGACAGCCTGTGGCGGTGCCTGGGTGGGGCTGC

TCCTCCAGGGCCACGTGCCAGGCCCGGGGCTGGCGGCTACTCAGCAGCCCTCC
TCACCCCGTCTGGGGTTCAGCCCCTCCTCTGCCACCTCCCCTATCCACCCAGCCC
ATTCTCTCTCCTGTCCAACCTAACCCCTTTCCTGCGGGCTTTTCCCCGGTCCCTTG
AGACCTCAGCCATGAGGAGTTGCTGTTTGTTTGACAAAGAAACCCAAGTGGGGGC
AGAGGGCAGAGGCTGGAGGCAGGGCCTTGCCCAGAGATGCCTCCACCGCTGCC
TAAGTGGCTGCTGACTGATGTTGAGGGAACAGACAGGAGAAATGCATCCATTCCT
CAGGGACAGAGACACCTGCACCTCCCCCCACTGCAGGCCCCGCTTGTCCAGCGC
CTAGTGGGGTCTCCCTCTCCTGCCTACTCACGATAAATAATCGGCCCACAGCTCC
CACCCCACCCCCTTCAGTGCCCACCAACATCCCATTGCCCTGGTTATATTCTCAC
GGGCAGTAGCTGTGGTGAGGTGGGTTTTCTTCCCATCACTGGAGCACCAGGCAC
GAACCCACCTGCTGAGAGACCCAAGGAGGAAAAACAGACAAAAACAGCCTCACA
GAAGAATATGACAGCTGTCCCTGTCACCAAGCTCACAGTTCCTCGCCCTGGGTCT
AAGGGGTTGGTTGAGGTGGAAGCCCTCCTTCCACGGATCCATGTAGCAGGACTG
AATTGTCCCCAGTTTGCAGAAAAGCACCTGCCGACCTCGTCCTCCCCCTGCCAGT
GCCTTACCTCCTGCCCAGGAGAGCCAGCCCTCCCTGTCCTCCTCGGATCACCGA
GAGTAGCCGAGAGCCTGCTCCCCCACCCCCTCCCCAGGGGAGAGGGTCTGGAG
AAGCAGTGAGCCGCATCTTCTCCATCTGGCAGGGTGGGATGGAGGAGAAGAATT
TTCAGACCCCAGCGGCTGAGTCATGATCTCCCTGCCGCCTCAATGTGGTTGCAAG
GCCGCTGTTCACCCACAGGGCTAAGAGCTAGCGCTGCCGCACCCCAGAGTGTGG
GAAGGGAGAGCGGGGCAGTCTCGGGTGGCTAGTCAGAGAGAGTGTTTGGGGGT
TCCGTGATGTAGGGTAAGGTGCCTTCTTATTCTCACTCCACCACCCAAAAGTCAAA
AGGTGCCTGTGAGGCAGGGGCGGAGTGATACAACTTCAAGTGCATGCTCTCTGC
AGCCAGCCCAGCCCAGCTGGTGGGAAGCGTCTGTCCGTTTACTCCAAGGTGGGG
TCTTTGTGAGAGTGAGCTGTAGGTGTGCGGGACCGGTACAGAAAGGCGTTCTTC
GAGGTGGATCACAGAGGCTTCTTCAGATCAGTGCTTGAGTTTGGGGAATGCGGC
CGCATTCCCTGAGTCACCAGGAATGTTAAAGTCAGTGGGAACGTGACTGCCCCAA
CTCCTGGAAGCTGTGTCCTTGCACCTGCATCCGTAGTTCCCTGAAAACCCAGAGA
GGAATCAGACTTCACACTGCAAGAGCCTTGGTGTCCACCTGGCCCCATGTCTCTC
AGAATTCTTCAGGTGGAAAAACATCTGAAAGCCACGTTCCTTACTGCAGAATAGCA
TATATATCGCTTAATCTTAAATTTATTAGATATGAGTTGTTTTCAGACTCAGACTCCA
TTTGTATTATAGTCTAATATACAGGGTAGCAGGTACCACTGATTTGGAGATATTTAT
GGGGGGAGAACTTACATTGTGAAACTTCTGTACATTAATTATTATTGCTGTTGTTAT
TTTACAAGGGTCTAGGGAGAGACCCTTGTTTGATTTTAGCTGCAGAACGTATTGGT
CCAGCTTGCTCTTCAGTGGGAGAAAACACTTGTAAGTTGCTAAACGAGTCAATCC
CCTCATTCAGGAAAACTGACAGAGGAGGGCGTGACTCACCCAAGCATATATAACT
AGCTAGAAGTGGGCCAGGACAGGCCCGGCGCGGTGGCTCACGCCTGTAATCCC
AGCAGTTTGGGAGGTCGAGGTAGGTGGATCACCTGAGGTCGGGAGTTCGAGACC

AACCTGACCAACATGGAGAAACCCTGTCTCTATTAAAAATACAAAAAAAAAAAAAA
AAAAAATAGCCGGGCATGGTGGCGCAAGCCTGTAATCCCAGCTACTCAGGAGGC
TGAGGCAGAAGAATTGAACCCAGGAGGTGGAGGTTGCAGTGAGCTGAGATCGTG
CCGTTACTCTCCAACCTGGACAACAAGAGCGAAACTCCGTCTTAGAAGTGGACCA
GGACAGGACCAGATTTTGGAGTCATGGTCCGGTGTCCTTTTCACTACACCATGTTT
GAGCTCAGACCCCCACTCTCATTCCCCAGGTGGCTGACCCAGTCCCTGGGGGAA
GCCCTGGATTTCAGAAAGAGCAAGTCTGGATCTGGGACCCTTTCCTTCCTTCCCT
GGCTTGTAACTCCACCAACCCATCAGAAGGAGAAGGAAGGAGACTCACCTCTGCC
TCAATGTGAATCAGACCCTACCCCACCACGATGTGGCCCTGGCCTGCTGGGCTCT
CCACCTCAGCCTTGGATAATGCTGTTGCCTCATCTATAACATGCATTTGTCTTTGT
AATGTCACCACCTTCCCAGCTCTCCCTCTGGCCCTGCCTTCTTCGGGGAACTCCT
GGAAATATCAGTTACTCAGCCCTGGGCCCCACCACCTAGGCCACTCCTCCAAAGG
AAGTCTAGGAGCTGGGAGGAAAAGAAAAGAGGGGAAAATGAGTTTTTATGGGGCT
GAACGGGGAGAAAAGGTCATCATCGATTCTACTTTAGAATGAGAGTGTGAAATAG
ACATTTGTAAATGTAAAACTTTTAAGGTATATCATTATAACTGAAGGAGAAGGTGCC
CCAAAATGCAAGATTTTCCACAAGATTCCCAGAGACAGGAAAATCCTCTGGCTGG
CTAACTGGAAGCATGTAGGAGAATCCAAGCGAGGTCAACAGAGAAGGCAGGAAT
GTGTGGCAGATTTAGTGAAAGCTAGAGATATGGCAGCGAAAGGATGTAAACAGTG
CCTGCTGAATGATTTCCAAAGAGAAAAAAAGTTTGCCAGAAGTTTGTCAAGTCAAC
CAATGTAGAAAGCTTTGCTTATGGTAATAAAAATGGCTCATACTTATATAGCACTTA
CTTTGTTGCAAGTACTGCTGTAAATAAATGCTTTATGCAAACCAA

Therefore, in a preferred embodiment of this aspect of the invention, the vitamin D receptor (VDR) agonist for use in the prevention, improvement, mitigation, or treatment of acute respiratory distress syndrome (ARDS) is selected from the list consisting of: calcifediol, calcitriol, or paricalcitol. In another preferred embodiment, other analogs are also used, preferably paricalcitol analogs, with the exception of hypercalcemia medications. Another, still much more preferred embodiment relates to calcifediol for use in the prevention, improvement, mitigation, or treatment of acute respiratory distress syndrome (ARDS). Still much more preferably, it relates to calcifediol for use in the improvement, mitigation, or treatment of acute respiratory distress syndrome (ARDS).

Cholecalciferol (vitamin D3) is a threshold nutrient among nutrients of the vitamin D endocrine system. The main source of vitamin D3 is endogenous synthesis in the skin; the intake of foods is usually a minor source. In circulation, vitamin D sterols are mainly transported by the vitamin D-binding protein, a serum glycoprotein secreted by the liver. Vitamin D itself is biologically inactive and is hydroxylated to 25-hydroxyvitamin D3 (25(OH)D3; calcifediol) predominantly in the liver. Furthermore, 1α-hydroxylation of 25(OH)D3 occurs mainly in the kidney and results in the formation of the active hormone VDES 1,25(OH)2D3 (calcitriol). The endocrine production of 1,25(OH)2D3 is rigorously regulated by bone and mineral metabolism parameters. For example, the production of 1,25(OH)2D3 is stimulated by the parathyroid hormone and inhibited by fibroblast growth factor 23 (FGF-23), for the main purpose of maintaining a suitable serum level of calcium and phosphorus and bone health, through the effects of 1,25(OH)2D3 in the kidney, intestine, and bone.

The effects of 1,25(OH)2D3 can be autocrine and paracrine because the expression of 25-hydroxyvitamin D-1α-hydroxylase (1α-hydroxylase) in multiple cells and tissues in addition to the kidney, including immune system cells, has been reported. This local 1,25(OH)2D3 tissue production seems to primarily depend on the availability of circulating 25(OH)D, but the production of 1,25(OH)2D is also regulated by other factors such as cytokines. Lastly, 1,25(OH)2D exerts its biological effects upon binding to the VDR that is virtually omnipresent in cells and tissues of the organism.

Furthermore, some antiviral agents used empirically to treat viral infection in patients affected by SARS-CoV-2 could interfere with the levels of enzyme CYP450. Protease inhibitors (PI) are potent inhibitors of liver enzymes CYP450. Treating resting macrophages with HIV PI antiretrovirals nelfinavir (NFV), indinavir (IDV), and ritonavir (RTV) also inhibits 25-hydroxylation and 1α-hydroxylation of vitamin D metabolites, consequently reducing the bioactivity of 1,25(OH)2D. While RTV shows the highest inhibition of CYP27B1, all Pls similarly inhibit CYP24A1 activity. In contrast, although it does not have a direct effect on enzymes CYP450, tenofovir (TFV), a nucleoside reverse transcriptase inhibitor (NRTI), is thought to mediate an indirect effect on vitamin D metabolism, through induced proximal renal tubular dysfunction, which could affect CYP27B1 activity and 1,25[OH]2D production. On the other hand, efavirenz (EFV), a non-nucleoside reverse transcriptase inhibitor (NNRTI), reduces serum levels of 25[OH]D even though it induces the expression of CYP24A1, which effectively limits any increase in 25[OH]D after vitamin D supplementation.

The inventors of the present invention indicate that patients admitted to the ICU for SARS due to SARS-CoV-2 undergoing treatment with these agents are at the risk of vitamin D deficiency induced or worsened by drugs, which may exacerbate the pre-existing deficiency associated with the infection.

Acute respiratory distress syndrome (ARDS) is a type of respiratory failure which is characterized by the rapid onset of widespread inflammation in the lungs. Symptoms include breathing distress (dyspnea), rapid breathing (tachypnea), and blue coloring of the skin (cyanosis). A decreased quality of life is common in those who survive.

The causes may include sepsis, pancreatitis, trauma, pneumonia (of various viruses and bacteria), and pulmonary aspiration. The underlying mechanism involves widespread damage to cells forming the barrier of the microscopic air sacs of the lungs, surfactant dysfunction, immune system activation, and dysfunction in the regulation of the body's blood clotting. Indeed, ARDS affects the ability of the lungs to exchange oxygen and carbon dioxide. The diagnosis in adults is based on the PaO₂/FiO₂ ratio (ratio of arterial oxygen at partial pressure to inspired oxygen fraction) of less than 300 mm Hg despite a positive end-expiratory pressure (PEEP) of more than 5 cm H₂O.

In another preferred embodiment of this aspect of the invention, the acute respiratory distress syndrome is due to a coronavirus infection, and more preferably, it is severe acute respiratory syndrome coronavirus 2.

Coronaviruses are organisms of the Superkingdom *Virus,* order *Nidovirales,* suborder *Cornidovirineae,* Family *Coronaviridae;* Subfamily *Orthocoronavirinae*; Genus *Betacoronavirus;* Subgenus *Sarbecovirus;* Species *Severe acute respiratory syndrome-related corona virus.*

In another preferred embodiment, 2 capsules containing 266 µg of calcifediol are administered at the start of the treatment, and 1 capsule is administered on days 3, 7, 15, and 30.

### PHARMACEUTICAL COMPOSITION AND DOSAGE FORM OF THE INVENTION

Another **aspect** of the invention relates to a composition comprising a vitamin D receptor (VDR) agonist, and/or a compound of formula (I) as previously defined, hereinafter composition of the invention, for the prevention, improvement, mitigation, or treatment of acute respiratory distress syndrome (ARDS)

In another preferred embodiment, the composition of the invention is a pharmaceutical composition. More preferably, the composition of the invention further comprises pharmaceutically acceptable adjuvants and/or vehicles. In another preferred embodiment, the composition of the invention further comprises another active ingredient.

Preferably, the other active ingredient is selected from an antiviral, chloroquine, hydroxychloroquine, IL-6 antagonists such as Tocilizumab, or any combinations thereof. More preferably, the antiviral is selected from Favilavir, Remdesivir, Galidesivir, Lopinavir, Ritonavir, Ribavirin, Darunavir, Cobicistat, or any combinations thereof

Preferably, for the prevention, improvement, mitigation, or treatment of acute respiratory distress syndrome, the other active ingredient is chloroquine, hydroxychloroquine, and/or IL-6 antagonists such as Tocilizumab.

The pharmaceutically acceptable adjuvants and vehicles that can be used in said compositions are the adjuvants and vehicles known to those skilled in the art and commonly used in preparing therapeutic compositions.

In the sense used in this description, the expression "therapeutically effective amount" refers to the amount of the agent or compound capable of carrying out the therapeutic action determined by its pharmacological properties, calculated to produce the desired effect, and it will generally be determined, among other causes, by the characteristics of the compound themselves, including the age and condition of the patient, the severity of the dysfunction or disorder, and the route and frequency of administration.

The compounds described in the present invention, the salts, prodrugs, and/or solvates thereof, as well as the pharmaceutical compositions containing them can be used together with other additional drugs or active ingredients to provide a combination therapy. Said additional drugs can be part of the same pharmaceutical composition or can alternatively be provided in the form of a separate composition that may or may not be administered simultaneously with respect to the pharmaceutical composition of the invention.

Another **aspect** of the invention relates to a dosage form, hereinafter dosage form of the invention, comprising a compound of the invention (a vitamin D receptor agonist), or the composition of the invention.

In this specification, "dosage form" is understood to mean the mixture of one or more active ingredients with or without additives having physical characteristics for the suitable dosing, storage, administration, and bioavailability thereof.

In another preferred embodiment of the present invention, the pharmaceutical compositions and dosage forms of the invention are suitable for oral administration in solid or liquid form. The possible forms for oral administration are tablets, capsules, syrups, or solutions, and they may contain conventional excipients known in the pharmaceutical sector, such as binding agents (e.g. syrup, acacia, gelatin, sorbitol, tragacanth, or polyvinylpyrrolidone), fillers (e.g. lactose, sugar, corn starch, calcium phosphate, sorbitol, or glycine), disintegrants (e.g. starch, polyvinylpyrrolidone, or microcrystalline cellulose) or a pharmaceutically acceptable surfactant such as sodium lauryl sulfate. Other dosage forms can be colloidal systems, among which polymeric nanoparticles, nanoemulsions, and nanocapsules are included.

Compositions for oral administration can be prepared by conventional Galenical Pharmacy methods, such as mixing and dispersion. The tablets can be coated following methods known in the pharmaceutical industry.

The pharmaceutical compositions and dosage forms can be adapted for parenteral administration, as sterile solutions, suspensions, or lyophilisates of the products of the invention, using the suitable dose. Suitable excipients, such as pH buffering agents or surfactants, can be used.

The formulations mentioned above can be prepared using conventional methods, such as those described in the Pharmacopeias of different countries and in other reference texts.

The term "medicinal product", as it is used in this specification, refers to any substance used for the prevention, diagnosis, mitigation, treatment, or curing of diseases in humans and in animals.

The administration of the compounds, compositions, or dosage forms of the present invention can be performed by means of any suitable method, such as intravenous infusion and oral, topical, or parenteral routes. Oral administration is preferred for patient convenience.

The administered amount of a compound of the present invention will depend on the relative efficacy of the chosen compound, the severity of the disease to be treated, and the weight of the patient. However, the compounds of this invention will be administered one or more times a day, for example 1, 2, 3, or 4 times daily, with a total dose between 0.1 and 1000 mg/Kg/day. It is important to take into account that it may be necessary to vary the dose, depending on the age and the condition of the patient, and vary the route of administration.

In a particular embodiment of the invention, 266 µg of calcifediol are administered according to the following dosage: 2 capsules at the start, and 1 capsule on days: 3, 7, 15, and 30.

The compounds and compositions of the present invention can be used together with other medicinal products in combined therapies. The other drugs may be part of the same composition or of another different composition for administration at the same time or at different times.

### COMBINED PREPARATION OF THE INVENTION

Therefore, another **aspect** of the invention relates to a combined preparation, hereinafter combined preparation of the invention, comprising:
a) A compound A, selected from a VDR receptor agonist, a compound of formula (I), and more preferably from calcitriol, calcifediol, or paricalcitol, or any combinations thereof, and
b) A compound B selected from an antiviral, chloroquine, hydroxychloroquine, IL-6 antagonists such as Tocilizumab, or any combinations thereof.

A preferred embodiment of this aspect relates to the combined preparation of the invention wherein the antiviral is selected from Favilavir, Remdesivir, Galidesivir, Lopinavir, Ritonavir, Ribavirin, Darunavir, Cobicistat, or any combinations thereof.

In another preferred embodiment of this aspect, compound A is calcifediol.

Another **aspect** relates to the use of the combined preparation of the invention for use as a medicinal product, or alternatively, for use in therapy.

Another **aspect** relates to the combined preparation of the invention for the combined administration thereof separately, simultaneously, or sequentially for the prevention, improvement, mitigation, or treatment of acute respiratory distress syndrome. It more preferably relates to the combined preparation of the invention for the combined administration thereof separately, simultaneously, or sequentially for the improvement, mitigation, or treatment of acute respiratory distress syndrome, wherein compound A is calcifediol.

Preferably, for the prevention, improvement, mitigation, or treatment of acute respiratory distress syndrome, the other active ingredient is chloroquine, hydroxychloroquine, and/or IL-6 antagonists such as Tocilizumab.

VDR agonists have favorable effects while significantly suppressing the inflammatory processes which change the immune response from the T helper 1 (Th1) domain to the T helper 2 (Th2) domain and offset the self-potentiating inflammatory circuit between immune and resident cells, especially due to the drop in the release of cytokines.

The metabolic enzymes of VDR and vitamin D are present in virtually all cells of the innate and adaptive branches of the immune system.

### Innate immune system:

Once activated, the VDR regulates the innate immune responses upstream and downstream of the signalling of the pattern recognition receptor activating the transcription of several genes encoding the nucleotide oligomerization domain protein of pattern recognition receptor 2 (NOD2), Toll-like receptor cofactor CD14, cathelicidin antimicrobial peptides (CAMP, LL-37) and DEFB4 / HBD2, as well as multiple cytokines, chemokines, and other signalling molecules.

### Adaptive immunity

VDR is present in T-lymphocytes, and calcitriol (1,25(OH)2D) is a T-cell proliferation and activation inhibitor. T-lymphocytes are made up of several subsets, including CD4+ helper T-cells, CD8+ cytotoxic T-cells, regulatory T-cells (Treg), natural killer cells, γδ T-cells, and memory cells. The effects of vitamin D signalling on T-cell subtypes have been extensively reviewed elsewhere.

VDR stimulation acts to suppress inflammation driven by T-cells and to improve the effects of suppressor Treg-cells. Communication between antigen presenting dendritic cells (DC) and T-cells seems to be particularly important in this regard. The intracrine production of 1,25(OH)2D induces a more tolerogenic DC phenotype characterized by the production of IL-10, which stimulates the production of Treg-cells.

Vitamin D metabolism and signalling in the acquired immune system. In antigen presenting cells (included DCs), 1,25(OH)2D3 inhibits surface expression of the major histocompatibility complex II (MHC-II), the antigen complex, and costimulatory molecules, in addition to the production of cytokines IL-12 and IL-23, indirectly shifting T-cell polarization from a Th1 and Th17 phenotype to a Th2 phenotype. Furthermore, 1,25(OH)2D3 directly affects T-cell responses by inhibiting the production of Th1 cytokines (IL-2 and IFN-γ) and Th17 cytokines (IL-17 and IL-21), as well as by stimulating the production of Th2 cytokine (IL-4). Furthermore, 1,25(OH)2D3 favors the development of Treg-cells by means of modulating DCs and by directly targeting T-cells. Lastly, 1,25(OH)2D3 blocks the differentiation of plasma cells, the production of IgG and IgM, and the proliferation of B cells.

VDR stimulation acts to significantly reduce IFN-α, IFN-γ, IL-1β, IL-6, IL-12, IL-18, IL-33, TNF-α, TGFβ, etc.) and chemokines (CCL2, CCL3, CCL5, CXCL8, CXCL9, CXCL10.

VDR agonists (25OHD3 (calcifediol), 1,25(OH)2D3 (calcitriol), 1αOHD3, and other calcitriol analogs with low calcemic inducing activity, such as paricalcitol for example) exert significant suppression of inflammatory processes which change the immune response from T helper 1 (Th1) to T helper 2 (Th2) to dominate and offset the self-sustaining inflammatory circuit between immune cells and resident cells, especially by means of a decline in the release of cytokines. Those molecules can, in fact, reduce the release of the 10 kDa interferon (IFN)-induced protein, IP-10/CXCL10, a potent chemokine driving Th1-mediated inflammation, as occurs in SARS, so it can be used in the treatment thereof

### Other mechanisms

The coronavirus S protein has been reported as being a significant determinant of the entry of the virus in the host cells. Like SARS-CoV, SARS-CoV-2 requires the angiotensin-converting enzyme 2 (ACE2) receptor to enter the cells. The binding of the virus with the host cell receptors is a significant determinant for the pathogenesis of the infection. SARS-CoV probably originated in bats and adapted to ACE2 variants when it crossed species to infect humans.

Furthermore, SARS-CoV down-regulates expression of the ACE2 protein in a replication-dependent manner, which would imply that the loss of ACE2 function can develop during SARS-CoV-2 infection. Given that ACE2 is a key player in the renin-angiotensin system (RAS), the loss of ACE2 function may have serious consequences for patients.

RAS, to which the Ang I (ACE) and ACE2 converting enzyme belong, is a complex network that plays an important role in various biological functions, including regulation of blood pressure and water balance. ACE cleaves Ang I into Ang II, while ACE2, a homolog of ACE, works like an endogenous counter-regulator of ACE by hydrolyzing Ang II into Ang-1-7. By binding to the Ang II type 1 receptor (AT1R), Ang II causes vasoconstriction, inflammation, and apoptosis, and Ang-(1-7) is resistant to the effects of Ang II by interacting with its own receptor. The balance between levels of ACE and ACE2 affects the endogenous Ang II:Ang-(1-7) ratio.

The vitamin D endocrine system modulates the expression of RAS. In mice, eliminating the vitamin D receptor or the 1α-hydroxylase gene (which converts 25[OH]D to 1,25[OH]D) increases the activity of RAS and induces hypertension and the treatment of said mice with 1,25-dihydroxyvitamin D suppresses the activity of RAS.

Then, calcitriol, calcifediol acting as a substrate for the auto/paracrine or endocrine synthesis of calcitriol or other VDR agonists, such as paricalcitol, also modulates the expression of renin-angiotensin system (RAS) members, including angiotensin (Ang)-converting enzymes I (ACE and ACE2), renin, and Ang II. This means that calcitriol can exert protective effects on lung injury caused by SARS by at least partially regulating the balance between the expression of the RAS members and specifically modulating the expression of the ACE2 pulmonary receptor, SARS-CoV-2.

Calcitriol suppresses the expression of renin and Ang II in rat lung tissues treated with lipopolysaccharide and inhibits the effect of the lipopolysaccharide on the expression of ACE and ACE2.

Furthermore, the vitamin D receptor (VDR), which mediates the activities of 1,25(OH)2D3, protects against sepsis-induced acute lung injury by inhibiting the pathway of the angiopoietin-2-TEK receptor tyrosine kinase-myosin light chain.

### Role of calcifediol in the protection against ARDS

The vitamin D endocrine system-mediated pathway has a potential function in the protection against ARDS. The pulmonary epithelium covers the entire bronchial and alveolar surface of the lung, and VDR is highly expressed in those lung cells which the epithelial cells of the airways constitutively convert inactive 25-dihydroxyvitamin D3 into active 1,25-dihydroxyvitamin D3. Active vitamin D generated by the pulmonary epithelium leads to a higher expression of genes regulated by vitamin D with important immune functions in the lung.

*VDR is highly expressed in lung cells in subjects with acute lung injury.*

It has also been demonstrated that VDR knock-out mice experienced LPS-induced acute lung injury of greater severity, which may primarily occur due to the degeneration of the close alveolar epithelial bonds through the reduced expression of occludin and zonula occludens-1. Paricalcitol, a non-hypercalcemic VDR analog, improved the LPS-induced acute lung injury or SARS by maintaining the alveolar barrier function.

As it is used herein, the term "active substance", "pharmaceutically active substance", "active ingredient" or "pharmaceutically active ingredient" means any component potentially providing pharmacological activity or another different effect on the diagnosis, cure, mitigation, treatment, or prevention of a disease, or affecting the structure or function of the human body or the body of other animals. The term includes those components which promote a chemical change in the preparation of the drug and are present therein in a modified form intended that provides the specific activity or effect.

It should be emphasized that the term "combined preparation", also referred to as a "juxtaposition", herein means that the components of the combined preparation do not need to be present bound to one another, for example in a true composition, to be available for the combined, separate, or sequential application thereof. The expression "juxtaposed" thereby implies that it is not necessarily a true combination given the physical separation of the components.

The composition or combined preparation of the invention can be administered to a subject suffering from said pathologies by means of any of the following routes: intraperitoneal, intravenous, intramuscular, subcutaneous, intrathecal, intraventricular, oral, enteral, parenteral, intranasal, or dermal. In a preferred embodiment, the routes of administration are preferably the intravenous route and the oral route.

Both the compositions of the present invention as well as the combined preparation or dosage forms of the invention can be formulated for administration to an animal, and more preferably to a mammal, including humans, in a variety of forms known in the state of the art. In that sense, they can be, with limitation, in a sterile aqueous solution or in biological fluids, such as serum. The aqueous solutions may or may not be buffered and have additional active or inactive components. The additional components include salts to modulate the ionic strength, preservatives including, but not limited to, antimicrobial agents, antioxidants, chelating agents, and the like, and nutrients including glucose, dextrose, vitamins, and minerals. Alternatively, the compositions can be prepared for administration in solid form. The compositions can be combined with several inert vehicles or excipients, including but not limited to: binders such as microcrystalline cellulose, tragacanth gum, or gelatin; excipients such as starch or lactose; dispersing agents such as alginic acid or corn starch; lubricants such as magnesium stearate, glidants such as colloidal silicon dioxide; sweetening agents such as sucrose or saccharin; or flavoring agents such as mint or methyl salicylate.

Such compositions or combined preparations and/or formulations thereof can be administered to an animal, including a mammal and, therefore, a human, in a variety of ways, including, but not limited to, intraperitoneal, intravenous, intramuscular, subcutaneous, intrathecal, intraventricular, oral, enteral, parenteral, intranasal or dermal.

The dosage for obtaining a therapeutically effective amount depends on a variety of factors, such as, for example, the age, weight, sex, tolerance, etc. of the mammal. In the sense used in this description, the expression "therapeutically effective amount" refers to the amount comprising the active ingredient or ingredients of the invention which produce the desired effect, and it will generally be determined, among other causes, by the characteristics typical of said prodrugs, derivatives, or analogs and the therapeutic effect to be achieved. The "adjuvants" and "pharmaceutically acceptable vehicles" which can be used in said compositions are the vehicles known to those skilled in the art.

Throughout the description and claims, the word "comprises" and its variants do not intend to exclude other technical features, additives, components, or steps. For those skilled in the art, other objects, advantages, and features of the invention will be inferred in part from the description and in part from putting the invention into practice. The following examples and drawings are provided by way of illustration and are not intended to be limiting of the present invention

### EXAMPLES OF THE INVENTION

### EXAMPLE 1. PRELIMINARY TRIAL: PILOT, PARALLEL, OPEN, RANDOMIZED, DOUBLE-BLIND CLINICAL TRIAL.

Preliminary study performed from 10 March 2020 on 72 patients admitted in pneumology department at Hospital Universitario Reina Sofia in Cordoba, according to hospital protocol.
Age 52.40 +/- 11.13 (21-73).
Men: n = 40, age 54.45 +/- 9.98
Women: n = 32, age 49.84 +/- 12.09
No age differences (p=0.081).

### ICU:

Yes: 14
No: 58

### Administration of vitamin D (calcifediol):

- Yes: 42 (58.3%).
- No: 30 (41.7%).

### ICU / Vit D

| | calcifediol YES | calcifediol NO | |
|---|---|---|---|
| ICU YES | 1 | 13 | 14 |
| ICU NO | 41 | 17 | 58 |
| | 42 | 30 | 72 |

The difference is statistically significant: Chi-squared with Yates' correction p=0.0001.

As shown in the results, progression of the disease requiring admission to the ICU occurred in only 1 of all the patients who were treated with calcifediol.

### EXAMPLE 2. RETROSPECTIVE COHORT STUDY IN 5 HOSPITALS IN SOUTHERN SPAIN

The administration of calcifediol in patients with COVID-19 reduces the development of SARS and the worsening of the various phases of the syndrome. It reduces ICU admissions and death by process by at least 25%. It also reduces days hospitalized, facilitating the recovery from hospitalization, acting significantly and positively in any of the phases throughout the natural history of the disease.

It is a treatment that has been widely used in clinical practice, and it is safe, inexpensive, and potentially highly effective given its low cost, having highly efficient cost-benefit impact on the prevention, improvement, mitigation, and treatment of SARS.

### Inclusion Criteria

1. Age ≥ 18 and < 90 years
2. Diagnosis confirmed by means of COVID-19 PCR
3. Radiological image compatible with inflammatory pleuropulmonary exudate
4. Signed direct or delegated informed consent.

### Exclusion Criteria

1. Intolerance or allergic to calcifediol or its components
2. Pregnancy

Patients can withdraw at any time throughout the entire study for whatever reason and without this affecting the medical treatment they receive in the future.

Primary objective: In patients 18> years <90, positive for coronavirus, recently hospitalized with or without conventional oxygen respiratory or non-invasive ventilatory support (such as BIPAP), treatment with Calcifediol will reduce the need for invasive ventilation and admissions to the Intensive Care Unit and deaths.

Primary efficacy marker: To evaluate improvement in the Sat O2 or PaO2/FiO2 ratio at 24 hours, at 72 hours, at 7 days, at 14 days, and at 28 days

### Secondary objectives

- Treatment with calcifediol in recently hospitalized patients with non-invasive ventilation reduces the time (in days) to discharge and coronavirus clearance.
- In patients who, over the course of the process, require invasive ventilation and admission to the ICU, the number of days until the removal of invasive ventilation, because the disease has improved will decrease, and the time (in days) to discharge from the ICU and clearance will decrease.
- Treatment with calcifediol in hospitalized patients improves clinical evolution.
- Treatment with calcifediol in hospitalized patients improves the evolution of blood count and biochemistry parameters routinely followed in the process.
- Treatment with calcifediol in hospitalized patients reduces inflammation markers: cytokines and chemokines involved in the development and maintenance of the syndrome.
- To confirm that low serum levels of 25OHD (<12 or <20 ng/ml), which facilitate the development of cytokine and chemokine storm, play a role in the negative progression to SARS in patients (especially in cases of younger patients and patients without risk factors).

### Secondary efficacy markers:

- To evaluate the improvement in the degree of dyspnea at 72 hours and at 7 days according to the Likert scale or the like (-3 to +3).
- To evaluate the improvement of the radiological findings by simple radiology at 72 hours and at 7 days.
- To evaluate the improvement transcutaneous O₂ saturation at 24 hours, at 72 hours, and at 7 days.
- To evaluate the improvement in the PaO₂.
- To measure the decrease in mortality (raw monthly rate).

### Design

Multicenter, open clinical trial in several hospitals in Andalusia, with 1:1 randomization. Two groups, both with the best available therapy:
Group 1) Medicinal product or substance under study: Calcifediol caps. 266 µg. Dosage Start: 2 capsules; days: 3, 7, 15, and 30: 1 capsule.
Group 2) Without calcifediol.

### Results

A non-randomized, retrospective, multicenter, open cohort study has been conducted on patients with COVID-19 confirmed by a laboratory between 14 February and 5 May 2020 in 5 hospitals in Andalusia, where a total of 537 patients were hospitalized with COVID-19 diagnosis (317 men [59%], mean age, 67.1 years), and 79 (14.7%) received treatment with calcifediol. In general, intrahospital mortality was 18.4%, with a mean of 10.5 days hospitalized. The death OR for patients who received calcifediol (mortality rate of 5.1%) was 0.20 (95% Cl, 0.07 to 0.57), compared with patients who did not receive said treatment (mortality rate of 20.7%; p <0.001). Patients who received calcifediol after admission were more likely than those who did not receive the treatment to present some comorbidity, but a lower rate, of heart failure, chronic obstructive pulmonary disease, and chronic kidney disease. In fitted Cox proportional hazards models, there were significant differences in mortality of patients who received calcifediol compared with patients who did not receive calcifediol [HR 0.17 (95% Cl: 0.05 to 0.51)]. This data indicates that treatment with calcifediol reduces mortality in patients hospitalized for COVID-19.

## Claims

1. A compound of formula (II): 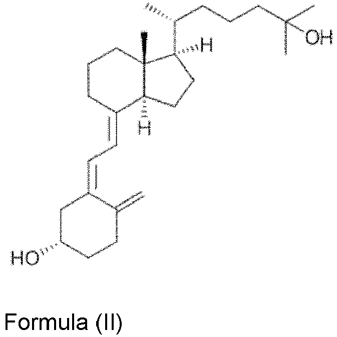 or any of the pharmaceutically acceptable salts, esters, tautomers, solvates, and hydrates thereof, or any combinations thereof, for the prevention, improvement, mitigation, or treatment of acute respiratory distress syndrome.

2. A composition comprising a compound as defined in claim 1, for use in the prevention, improvement, mitigation, or treatment of acute respiratory distress syndrome.

3. The composition for use according to the preceding claim, wherein said composition comprises at least one additional active ingredient.

4. The composition for use according to claim 3, wherein said additional active ingredient is selected from the list consisting of an antiviral, chloroquine, hydroxychloroquine, an IL-6 antagonist, or any combinations thereof.

5. A combined preparation comprising:
a) A compound as defined in claim 1, or a composition according to any of claims 2-4, and
b) A composition comprising an additional active ingredient selected from the list consisting of an antiviral, chloroquine, hydroxychloroquine, an IL-6 antagonist, or any combinations thereof.

6. The combined preparation according to claim 5 for the combined administration thereof separately, simultaneously, or sequentially for the prevention, improvement, mitigation, or treatment of acute respiratory distress syndrome.

7. The compound, the composition, or the combined preparation for use according to any of claims 1-6, wherein the acute respiratory distress syndrome is due to a coronavirus infection.

8. The compound, the composition, or the combined preparation for use according to any of claims 1-7, wherein the acute respiratory distress syndrome is severe acute respiratory syndrome coronavirus 2.

9. The compound, the composition, or the combined preparation for use according to any of claims 1-8, wherein 2 capsules containing 266 µg of the compound of formula (II) (calcifediol) are administered at the start of the treatment, and 1 capsule is administered on days 3, 7, 15, and 30.

10. The composition or the combined preparation for use according to any of claims 2-9, wherein the antiviral is selected from Favilavir, Remdesivir, Galidesivir, Lopinavir, Ritonavir, Ribavirin, Darunavir, Cobicistat, or any combinations thereof.

11. The composition or the combined preparation for use according to any of claims 2-10, wherein the antiviral is Remdesivir.
